# EUROPEAN PATENT APPLICATION

(11) **EP 2 722 073 A1**
(43) Date of publication of application: **23.04.2014**
(21) Application number: 13163258.0
(22) Date of filing: 11.04.2013
(51) Int. Cl.: A61N 2/00, A61N 2/06

(54) **Attachable magnetic acupuncture**

(30) Priority: 19.10.2012 KR 20120116656
(71) Applicant: Infopia Co., Ltd., Gyeonggi-do 431-080 (KR)
(72) Inventor: Bae, Byeong Woo, 431-080 Hogye-dong (KR); Park, Yong Kyu, 431-080 Hogye-dong (KR)
(74) Representative: Dantz, Jan Henning

(57) **Abstract**

Provided is an attachable magnetic acupuncture that is capable of reinforcing energy at meridians and acupoints of the human body by using the principle of electromagnetic conduction. The attachable magnetic acupuncture includes a plurality of magnets respectively disposed at vertexes of a regular polygonal shape so that magnetic force between the magnets adjacent to each other is minimized, and an encapsulation layer encapsulating the plurality of magnets.

## Description

### BACKGROUND OF THE INVENTION

### 1. Field of the Invention

The prevent invention relates to an attachable magnetic acupuncture, and more particularly, to an attachable magnetic acupuncture that is capable of reinforcing energy at meridians and acupoints of the human body by using the principle of electromagnetic conduction.

### 2. Description of the Related Art

Acupuncture therapies are being applied as major therapeutic techniques for traditional oriental medicines. Acupuncture therapies stimulate meridians that are energy channels within the human body and acupoints by using acupunctures to obtain treatment effects. Such an acupuncture therapy may have temporary effects, but cause painful sensitivity during the procedure.

Thus, magnetic therapies in which magnets instead of acupunctures are attached to meridians and acupoints of the human body to continuously stimulate the meridians and acupoints, thereby treating the human body and improving health are trying in recent years.

However, such a magnetic therapy may increase magnetic force to increase generation of energy, but have difficulty in reduction of the magnetic force that is harmful to the human body.

### SUMMARY OF THE INVENTION

The present invention provides an attachable magnetic acupuncture in which magnets are disposed in a regular polygonal shape to minimize magnetic force passing through a center between the magnets, thereby minimizing the magnetic force affecting the human body.

The technical objects of the present invention are not limited to those described above, and it will be apparent to those of ordinary skill in the art from the following description that the present invention includes other technical objects not specifically mentioned herein.

According to an aspect of the present invention, there is provided an attachable magnetic acupuncture including: a plurality of magnets respectively disposed at vertexes of a regular polygonal shape so that magnetic force between the magnets adjacent to each other is minimized; and an encapsulation layer encapsulating the plurality of magnets; and an adhesion layer surrounding the encapsulation layer.

### BRIEF DESCRIPTION OF THE DRAWINGS

The above and other features and advantages of the present invention will become more apparent by describing in detail exemplary embodiments thereof with reference to the attached drawings in which:
FIG. 1A is a schematic perspective view of an attachable magnetic acupuncture according to a first embodiment of the present invention;
FIG. 1B is a lower plan view of the attachable magnetic acupuncture according to the first embodiment of the present invention;
FIG. 1C is a cross-sectional side view of the attachable magnetic acupuncture according to the first embodiment of the present invention;
FIG. 2A is a schematic perspective view of an attachable magnetic acupuncture according to a second embodiment of the present invention;
FIG. 2B is a lower plan view of the attachable magnetic acupuncture according to the second embodiment of the present invention;
FIG. 2C is a cross-sectional side view of the attachable magnetic acupuncture according to the second embodiment of the present invention;
FIG. 3A is a view illustrating an example of a repulsive region between magnetic fields in the attachable magnetic acupuncture according to the first embodiment of the present invention;
FIG. 3B is a view illustrating an example of a repulsive region between magnetic fields in the attachable magnetic acupuncture according to the second embodiment of the present invention;
FIG. 4 is an image of magnetic force lines between an N pole and an S pole of an attachable magnetic acupuncture according to an embodiment of the present invention;
FIG. 5 is a view illustrating an example of Gaussian distribution for explaining a repulsive region between magnetic fields in the attachable magnetic acupuncture according to an embodiment of the present invention; and
FIG. 6 is a schematic view of an attachable magnetic acupuncture according to another embodiment of the present invention.

### DETAILED DESCRIPTION OF THE INVENTION

Preferred embodiments of the present invention will be described below in more detail with reference to the accompanying drawings, such that those skilled in the art can realizes the technical ideas of the present invention without difficulties. The present invention may, however, be embodied in different forms and should not be construed as limited to the embodiments set forth herein. In the drawings, anything unnecessary for describing the present disclosure will be omitted for clarity. Also, like reference numerals in the drawings denote like elements. Moreover, detailed descriptions related to well-known functions or configurations will be ruled out in order not to unnecessarily obscure subject matters of the present invention.

For reference, explaining a change of magnetic force and a flow of energy according to a disposition of a magnet, when permanent magnets having the same pole are adjacent to each other, a repulsive magnetic force may be generated between the permanent magnets having the same pole to push the permanent magnets against each other, thereby offsetting a portion of the magnetic force. However, since magnetic force coming out from an N pole comes again into an S pole, energy induced by the magnetic force is not offset. Thus, the energy induced by an increase of the magnetic force coming out from the N pole may increase.

Here, a direction of the energy may be determined by the N and S poles of the magnets. Thus, the energy of the human body may be reinforced or removed by using the N and S poles. However, when the human body undergoes a surgical procedure, a method for reinforcing energy of the human body is the safest. If the energy is removed, internal organs of the human body may be damaged. Also, while the rules of incompatibility and assistance of five elements that are used in oriental medical techniques are applied, if it is intended to remove a strong portion, the rule of incompatibility should be applied to perform a procedure for reinforcing objects that are incompatible against each other, for safety.

Thus, the present invention may provide an attachable magnetic acupuncture in which magnets are disposed to reinforce energy of the human body.

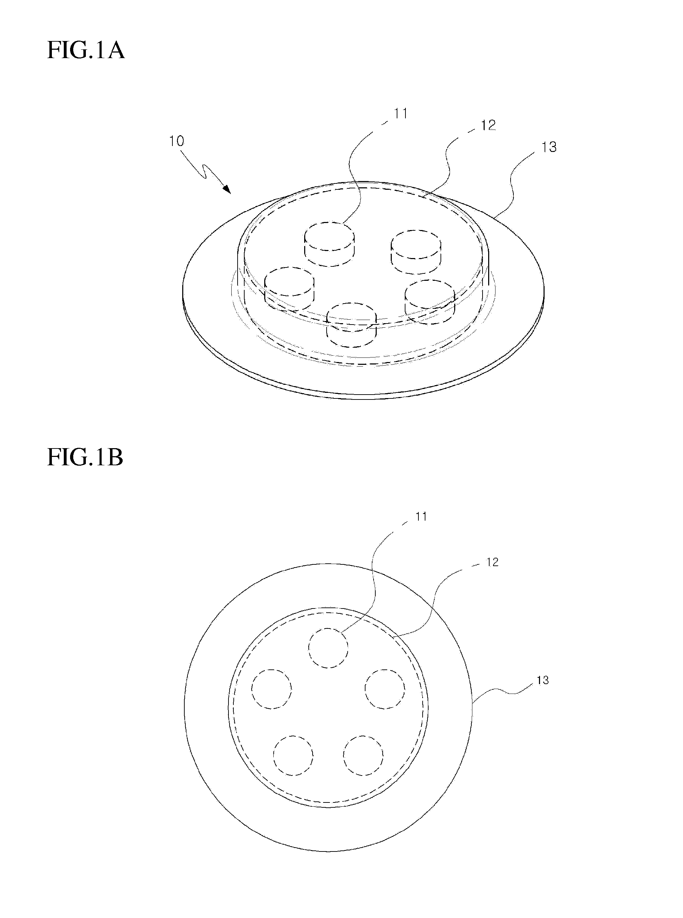

First, an attachable magnetic acupuncture according to a first embodiment of the present invention will be described with reference to FIGS. 1A to 1C. Here, in the attachable magnetic acupuncture according to the first embodiment of the present invention, magnets are disposed at vertexes of a regular pentagonal shape, respectively.

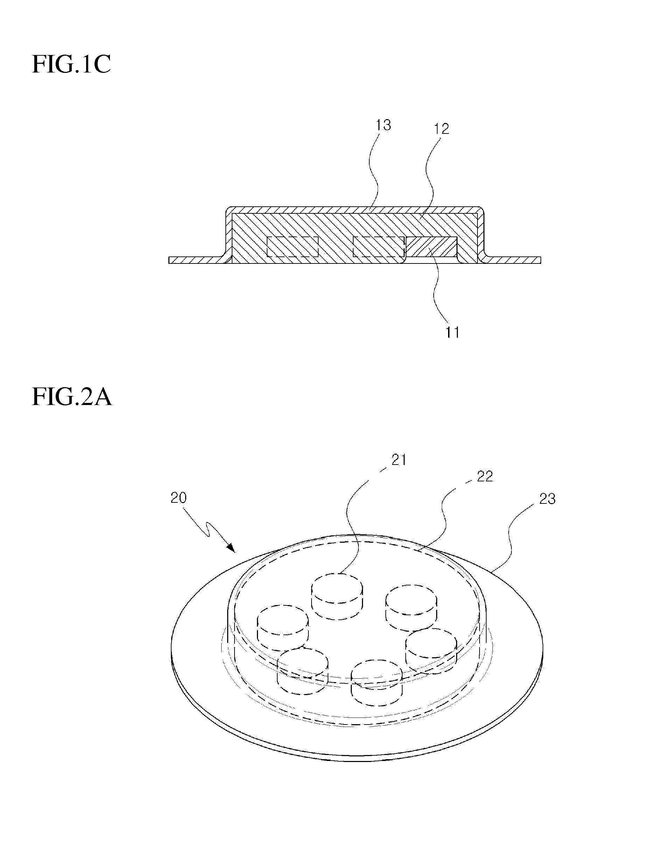

FIG. 1A is a schematic perspective view of an attachable magnetic acupuncture according to a first embodiment of the present invention, FIG. 1B is a lower plan view of the attachable magnetic acupuncture according to the first embodiment of the present invention, and FIG. 1C is a cross-sectional side view of the attachable magnetic acupuncture according to the first embodiment of the present invention.

Referring to FIGS. 1A to 1C, an attachable magnetic acupuncture 10 includes a plurality of magnets 11 respectively disposed at vertexes of a regular polygonal shape so that magnetic force between the magnets is minimized, an encapsulation layer 12 encapsulating the plurality of magnets 11, and an adhesion layer 13 surrounding the encapsulation layer 12 to provide an adhesion surface. Here, although each of the magnets 11, the encapsulation layer 12, and the adhesion layer 13 has a circular shape, the present invention is not limited thereto. For example, each of the magnets 11, the encapsulation layer 12, and the adhesion layer 13 may have a polygonal shape. That is, if each of the magnets 11, the encapsulation layer 12, and the adhesion layer 13 may provide its corresponding function, each of the magnets 11, the encapsulation layer 12, and the adhesion layer 13 may have various shapes.

Each of the magnets 11 may have a cylindrical shape with a predetermined thickness. The magnet 11 may be a permanent magnet in which lower and upper ends with respect to a rotation center axis of the cylinder may have S and N poles, respectively. The magnet 11 may have a thickness of about 2 mm or less and a diameter of about 2 mm or less. Also, the magnet 11 may be a permanent magnet having magnetic flux density of about 2,000 gauss or more.

The encapsulation layer 12 may be formed of silicon. The magnet 11 may be fixed by being inserted into the encapsulation layer 12.

The adhesion layer 13 may extend toward a side surface of the encapsulation layer 12 while surrounding the encapsulation layer 12 to provide the adhesion surface. The adhesion layer 13 may be replaceable.

Particularly, in the attachable magnetic acupuncture 10, the plurality of magnets 11 may be disposed at vertexes of a regular pentagonal shape of the encapsulation layer 12, respectively. That is, five magnets 11 may be respectively disposed on vertexes of the regular pentagonal shape on one surface of the encapsulation layer 12. Also, the magnet 11 may contact one surface of the encapsulation layer 12 so that the S pole of the magnet 11 is exposed, i.e., the N pole contacts one surface of the encapsulation layer 12. Here, a circumscribed circle of the regular pentagonal shape may have a diameter of about 10 mm or less. This is done for a reason in which, when a diameter of the circumscribed circle is larger than 10 mm, i.e., when the magnets are far away from a central portion of the regular pentagonal shape, repulsive force at the central portion may be reduced, and thus, it may be difficult to maximize an offsetting of the magnetic force at the central portion.

As described above, in the attachable magnetic acupuncture 10, the magnetic force may be reinforced through the disposition of the plurality of magnets to increase the generation of the energy induced by the magnetic force. In addition, magnetic force in a central region between the magnets adjacent to each other and a region connecting the central portion of the regular pentagonal shape to the central region between the magnets adjacent to each other may be substantially zero to minimize magnetic force passing through a center between the magnets 11, thereby minimizing magnetic force affecting the human body. Thus, the treatment effects at meridians or acupoints of the human body may be improved.

Also, the magnet 11 of the attachable magnetic acupuncture 10 may be buried in the encapsulation layer 12 so that the N pole faces an upper side, and the S pole faces a lower side, i.e., the S pole is disposed on a skin of the human body to perform a procedure. Thus, the magnetic force lines passing through the central portion of the regular pentagonal shape proceeds downward from an upper portion of the magnet 11 to reinforce the energy of the human body.

Also, the attachable magnetic acupuncture 10 may be semipermanently used by replacing only the adhesion layer 13.

The attachable magnetic acupuncture 10 may further include a base material (not shown) which is attached to bottom surfaces of the magnets 11 and the encapsulation layer 12 to prevent the magnets 11 from directly contacting the skin of the human body and being separated from the encapsulation layer 12.

Also, the attachable magnetic acupuncture 10 may further include a release paper (not shown) that is attached to the adhesion surface of the at least adhesion layer 13 so that the attachable magnetic acupuncture 10 is easily moved and stored.

Next, an attachable magnetic acupuncture according to a second embodiment of the present invention will be described with reference to FIGS. 2A to 1C. Here, in the attachable magnetic acupuncture according to the second embodiment of the present invention, magnets are disposed at vertexes of a regular hexagonal shape, respectively.

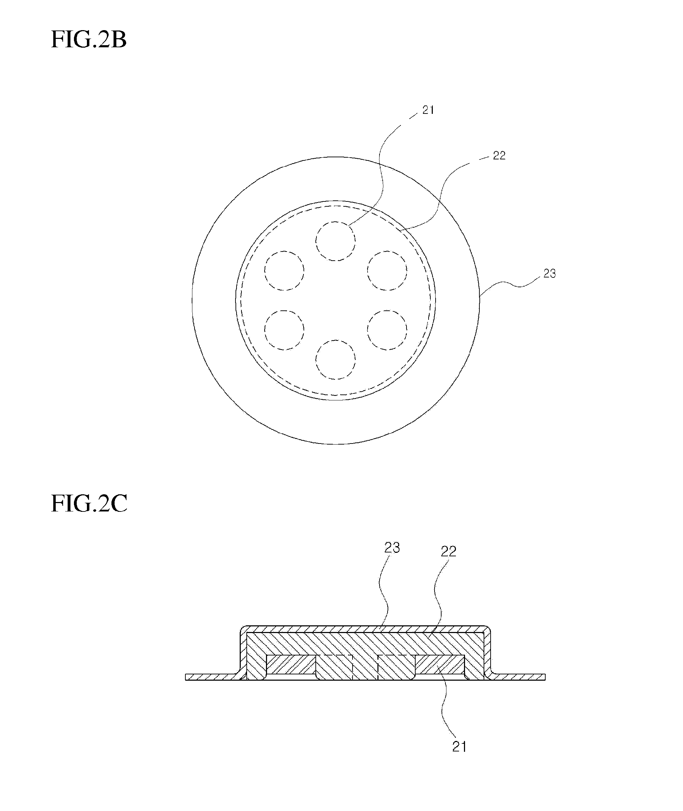

FIG. 2A is a schematic perspective view of an attachable magnetic acupuncture according to a second embodiment of the present invention. FIG. 2B is a lower plan view of the attachable magnetic acupuncture according to the second embodiment of the present invention, and FIG. 2C is a cross-sectional side view of the attachable magnetic acupuncture according to the second embodiment of the present invention.

Referring to FIGS. 2A to 2C, an attachable magnetic acupuncture 20 includes a plurality of magnets 21 respectively disposed at vertexes of a regular polygonal shape so that magnetic force between the magnets is minimized, an encapsulation layer 22 encapsulating the plurality of magnets 21, and an adhesion layer 23 surrounding the encapsulation layer 22 to provide an adhesion surface.

Each of the magnets 21 may have a cylindrical shape with a predetermined thickness. The magnet 21 may be a permanent magnet in which lower and upper ends with respect to a rotation center axis of the cylinder may have S and N poles, respectively. The magnet 21 may have a thickness of about 2 mm or less and a diameter of about 2 mm or less. Also, the magnet 21 may be a permanent magnet having magnetic flux density of about 2,000 gauss or more.

The encapsulation layer 22 may be formed of silicon. The magnet 21 may be fixed by being inserted into the encapsulation layer 22. That is, the magnet 22 may be buried in the encapsulation layer 22 so that an S pole of the magnet 22 is exposed.

The adhesion layer 23 may extend toward a side surface of the encapsulation layer 22 while surrounding the encapsulation layer 22 to provide the adhesion surface.

In the attachable magnetic acupuncture 20 according to the current embodiment, the plurality of magnets 21 may be disposed at vertexes of a regular hexagonal shape of the encapsulation layer 22, respectively. That is, six magnets 21 may be respectively disposed on vertexes of the regular hexagonal shape on one surface of the encapsulation layer 22. Also, the magnet 21 may contact one surface of the encapsulation layer 22 so that the S pole of the magnet 21 is exposed, i.e., the N pole contacts one surface of the encapsulation layer 22. Here, a circumscribed circle of the regular hexagonal shape may have a diameter of about 10 mm or less. This is done for a reason in which, when a diameter of the circumscribed circle is larger than 10 mm, i.e., when the magnets are far away from a central portion of the regular hexagonal shape, repulsive force at the central portion may be reduced, and thus, it may be difficult to maximize an offsetting of the magnetic force at the central portion.

As described above, in the attachable magnetic acupuncture 20, the magnetic force may be reinforced through the disposition of the plurality of magnets to increase the generation of the energy induced by the magnetic force. In addition, magnetic force in a central region between the magnets adjacent to each other and a region connecting the central portion of the regular pentagonal shape to the central region between the magnets adjacent to each other may be substantially zero to minimize magnetic force passing through a center between the magnets 21, thereby minimizing magnetic force affecting the human body. Thus, the treatment effects at meridians or acupoints of the human body may be improved.

Also, the magnet 21 of the attachable magnetic acupuncture 20 may be buried in the encapsulation layer 22 so that the N pole faces an upper side, and the S pole faces a lower side, i.e., the S pole contacts a skin of the human body to perform a procedure. Thus, the magnetic force lines passing through the central portion of the regular hexagonal shape proceeds downward from an upper portion of the magnet 21 to reinforce the energy of the human body.

The attachable magnetic acupuncture 20 may further include a base material (not shown) which is attached to bottom surfaces of the magnets 21 and the encapsulation layer 22 to prevent the magnets 21 from directly contacting the skin of the human body and being separated from the encapsulation layer 22.

Also, the attachable magnetic acupuncture 20 may further include a release paper (not shown) that is attached to the adhesion surface of the at least adhesion layer 23 so that the attachable magnetic acupuncture 20 is easily moved and stored.

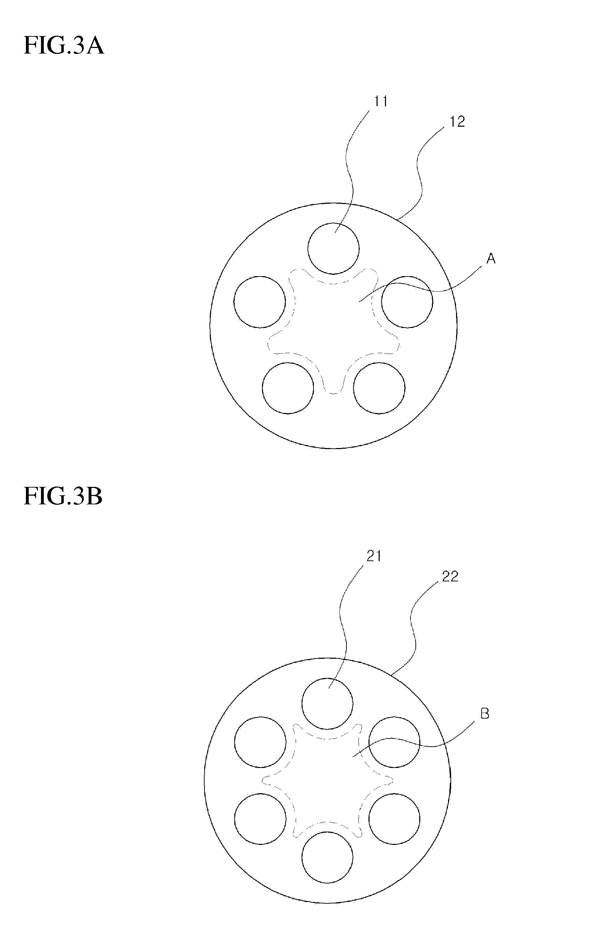

FIG. 3A is a view illustrating an example of a repulsive region between magnetic fields in the attachable magnetic acupuncture according to the first embodiment of the present invention.

Referring to FIG. 3A, when magnets 11 are disposed at vertexes of a regular pentagonal shape on one surface of an encapsulation layer 12, a region A in which magnetic force is minimized is generated in a central portion between magnets by an S pole of each of the magnets 11.

That is, repulsive magnetic force may occur in the central portion (the region A) between the magnets 11 having the regular pentagonal shape to minimize intensity of the magnetic force, and thus induced energy may maximally increase.

Thus, in the attachable magnetic acupuncture, the region A may be provided as a path through which the energy smoothly flows.

FIG. 3B is a view illustrating an example of a repulsive region between magnetic fields in the attachable magnetic acupuncture according to the second embodiment of the present invention.

Referring to FIG. 3B, when magnets 21 are disposed at vertexes of a regular hexagonal shape on one surface of an encapsulation layer 22, a region B in which magnetic force is minimized is generated in a central portion between magnets by an S pole of each of the magnets 21.

That is, repulsive magnetic force may occur in the central portion (the region B) between the magnets 21 having the regular hexagonal shape to minimize intensity of the magnetic force, and thus induced energy may maximally increase.

Thus, in the attachable magnetic acupuncture, the region B may be provided as a path through which the energy smoothly flows.

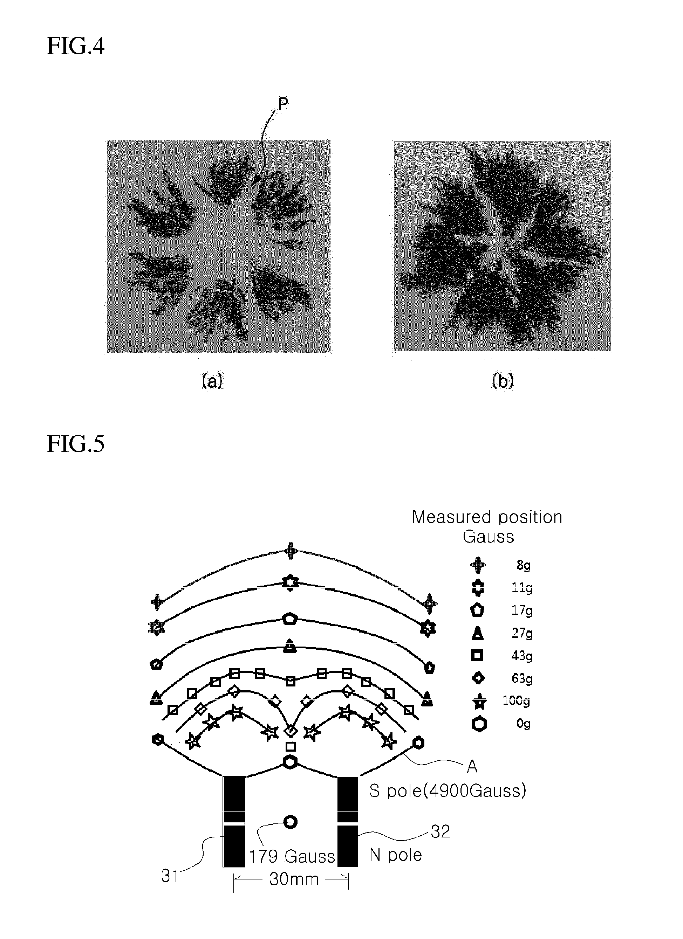

FIG. 4 is an image of magnetic force lines between an N pole and an S pole of an attachable magnetic acupuncture according to an embodiment of the present invention. FIG. 4A is an image illustrating distribution of magnetic force lines of an N pole, and FIG. 4B is an image illustrating distribution of magnetic force lines of an S pole.

Referring to FIGS. 4 A and 4B, when magnets are disposed in a regular hexagonal shape, it may be seen that repulsive force between magnets disposed on vertexes of the regular hexagonal shape to offset magnetic force lines in a region P connecting a central portion between the magnets adjacent to each other to a central portion of the hexagonal shape. Thus, the region P connecting the central portion between the adjacent magnets to the central portion of the hexagonal shape may provide a path through which energy flows. As a result, as magnetic force reaches substantially zero. The induced energy may be maximized.

Therefore, the number of magnets of the attachable magnetic acupuncture may increase to increase the magnetic force. In addition, the magnets may be disposed in regular pentagonal or hexagonal shape to maximize the offsetting of the magnetic force at the center between the magnets.

FIG. 5 is a view illustrating an example of Gaussian distribution for explaining a repulsive region between magnetic fields in the attachable magnetic acupuncture according to an embodiment of the present invention.

Referring to FIG. 5, when two permanent magnets are disposed at a distance of about 30 mm, it may be seen that line A indicates points at which a value of magnetic force between S poles of the two magnets is about zero in Gaussian distribution.

That is, when magnets are disposed in a regular pentagonal or hexagonal shape in the attachable magnetic acupuncture, a repulsive region in which the magnetic force is substantially zero occurs in a region connecting a central portion of the magnets adjacent to each other to a central portion of the regular pentagonal or hexagonal shape.

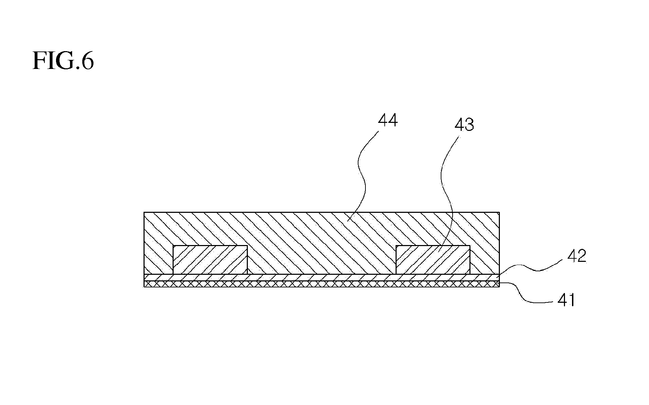

FIG. 6 is a schematic view of an attachable magnetic acupuncture according to another embodiment of the present invention.

Referring to FIG. 6, an attachable magnetic acupuncture includes a plurality of magnets 43 respectively disposed at vertexes in a regular polygonal shape on one surface of an adhesion layer 42 so that magnetic force between magnets is substantially zero, an encapsulation layer 44 encapsulating the plurality of magnets 43, and a release layer 41 attached on the other surface of the adhesion layer 42. Here, the regular polygonal shape may include a regular pentagonal or hexagonal shape.

Explaining constitutes of the attachable magnetic acupuncture, the magnet 43 may have a cylindrical shape with a thin thickness, but it is not limited thereto. For example, the magnet 43 may be a permanent magnet of which lower and upper ends with respect to a rotation center axis of the cylinder may have S and N poles, respectively. The magnet 43 may have a thickness of about 2 mm or less and a diameter of about 2 mm or less. Also, the magnet 43 may be a permanent magnet having magnetic flux density of about 2,000 gauss or more.

The magnet 43 may be disposed at each of vertexes of the regular pentagonal or hexagonal shape on one surface of the encapsulation layer 44. That is, five or six magnets 43 may be respectively disposed on vertexes of the one surface of the encapsulation layer 44. Also, an N pole of the magnet 43 may contact the one surface of the encapsulation layer 44 so that an S pole of the magnet 43 is exposed from the encapsulation layer 44. Here, a circumscribed circle of the regular pentagonal or hexagonal shape may have a diameter of about 10 mm or less. This is done for a reason in which, when a diameter of the circumscribed circle is larger than 10 mm, i.e., when the magnets are far away from a central portion of the regular polygonal shape, repulsive force at the central portion may be reduced, and thus, it may be difficult to maximize an offsetting of the magnetic force at the central portion.

The encapsulation layer 44 may be formed of silicon. The magnet 43 may be fixed by being inserted into the encapsulation layer 44.

Also, the adhesion layer 42 may be disposed on bottom surfaces of the magnet 43 and the encapsulation layer 44, and an adhesion surface may be provided on a surface opposite to the contact surface between the magnet 43 and the encapsulation layer 44.

The release layer 41 may be attached to the adhesion surface of the adhesion layer 42 so that the attachable magnetic acupuncture is easily moved and stored.

According to the present invention, the magnets may be disposed in the regular polygonal shape to minimize the magnetic force affecting the human body. Therefore, the treatment effects at the meridians or acupoints of the human body may be improved.

While the present invention has been particularly shown and described with reference to exemplary embodiments thereof, it will be understood by those of ordinary skill in the art that various changes in form and details may be made therein without departing from the spirit and scope of the present invention as defined by the following claims. The preferred embodiments should be considered in descriptive sense only and not for purposes of limitation, and all differences within the scope will be construed as being included in the present invention.

## Claims

1. An attachable magnetic acupuncture comprising:
a plurality of magnets respectively disposed at vertexes of a regular polygonal shape so that magnetic force between the magnets adjacent to each other is minimized; and
an encapsulation layer encapsulating the plurality of magnets.

2. The attachable magnetic acupuncture of claim 1, further comprising an adhesion layer surrounding the encapsulation layer, the adhesion layer extending from a side surface of the encapsulation layer to provide an adhesion surface.

3. The attachable magnetic acupuncture of claim 1, further comprising an adhesion layer,
wherein the plurality of magnets are disposed on one side of the adhesion layer, and wherein a bottom surface of the encapsulation layer is disposed on the one side of the adhesion layer.

4. The attachable magnetic acupuncture of claim 1, wherein a region connecting a central portion of the regular polygonal shape to a central portion between the adjacent magnets respectively disposed at the vertexes of the regular polygonal shape has magnetic force of about zero.

5. The attachable magnetic acupuncture of claim 1, wherein each of the plurality of magnets has a cylindrical shape, and
the magnets comprises a permanent magnet of which lower and upper ends in a rotation center axis direction of the cylinder have S and N poles, respectively,
wherein the S pole is exposed to the outside.

6. The attachable magnetic acupuncture of claim 1, wherein the regular polygonal shape comprises a regular pentagonal shape, and the number of the plurality of magnets is five.

7. The attachable magnetic acupuncture of claim 1, wherein the regular polygonal shape comprises a regular hexagonal shape, and the number of the plurality of magnets is six.

8. The attachable magnetic acupuncture of claim 1, wherein each of the plurality of magnets has a thickness of about 2 mm or less.

9. The attachable magnetic acupuncture of claim 8, wherein each of the plurality of magnets has a diameter of about 2 mm or less.

10. The attachable magnetic acupuncture of claim 9, wherein a circumscribed circle of the regular polygonal shape has a diameter of about 10 mm or less.

11. The attachable magnetic acupuncture of claim 1, wherein each of the plurality of magnets has magnetic flux density of about 2,000 gauss or more.

12. The attachable magnetic acupuncture of claim 1, wherein the encapsulation layer is formed of silicon.

13. The attachable magnetic acupuncture of claim 2, further comprising a base material attached to bottom surfaces of the plurality of magnets and the encapsulation layer.

14. The attachable magnetic acupuncture of claim 13, further comprising a release layer attached to the at least adhesion surface of the adhesion layer.

15. The attachable magnetic acupuncture of claim 3, further comprising a release layer attached to other side of the adhesion layer.
